Europäisches Patentamt

(19) European Patent Office

Office européen des brevets

(11) Publication number: **0 188 026**
**B1**

(12) **EUROPEAN PATENT SPECIFICATION**

<table>
<tr><td>(45) Date of publication of the patent specification:<br>30.05.90</td><td>(51) Int. Cl.⁵: <strong>A01N 37/40, A01N 25/34,</strong><br><strong>A61L 15/00</strong></td></tr>
</table>

(21) Application number: **85202089.0**

(22) Date of filing: **17.12.85**

⑤ **A01N 37/40, A01N 25/34,**
**A61L 15/00**
**// (A01N37/40, 31:02)**

(54) **Disinfactant compositions.**

(30) Priority: **03.01.85  GB 8500108**

(43) Date of publication of application:
**23.07.86 Bulletin 86/30**

(45) Publication of the grant of the patent:
**30.05.90 Bulletin 90/22**

(84) Designated Contracting States:
**AT BE CH DE FR GB IT LI NL SE**

(56) References cited:
**FR-A- 2 370 433**
**GB-A- 778 240**
**GB-A- 1 577 396**
**US-A- 1 715 251**
**US-A- 4 485 029**

(73) Proprietor: **UNILEVER NV, Burgemeester**
**s'Jacobplein 1 P.O. Box 760, NL-3000 DK Rotterdam(NL)**
(84) Designated Contracting States: **BE CH DE FR IT LI NL SE**
**AT**

(73) Proprietor: **UNILEVER PLC, Unilever House Blackfriars**
**P.O. Box 68, London EC4P 4BQ(GB)**
(84) Designated Contracting States: **GB**

(72) Inventor: **Groenewegen, Dirk, Lichtegaarde 30,**
**NL-3436 ZV Nieuwegein(NL)**

(74) Representative: **van Gent, Jan Paulus et al, Unilever N.V.**
**Patent Division P.O. Box 137, NL-3130 AC**
**Vlaardingen(NL)**

**Description**

The present invention relates to disinfectant compositions, and in particular to disinfectant compositions which comprise an aqueous solution of a lower alcohol and anti-microbial compound.

Disinfectant compositions are widely used for hard-surface disinfection in hospitals, large canteens, restaurants and the like. To meet the required level of disinfection, conventional products either comprise high percentages of alcohol, i.e. about 60% or more, or they comprise in addition to the alcohol an anti-microbial compound. The first type of product suffers from severe disadvantages due to the safety hazards (both during manufacturing and at application) connected with the high alcohol content and the corresponding low flash point of such products. In the second type of product the alcohol content is much lower, i.e. about 40%, but it proves necessary to include an anti-microbial compound to compensate for the decrease in alcohol content. In the majority of products, to this purpose aldehydes, in particular formaldehyde, are included, but, in view of their toxic properties, only low concentrations can be included and ther require severe caution in handling by the consumer. Other types of anti-microbial compounds are e.g. quaternary ammonium compounds, but their suitability is rather limited owing to their tendency to cause residues on the surface to be treated.

The US-patent 1 715 251 discloses disinfactant compositions containing an alkyl ester of m- or p-hydroxybenzoic acid dissolved in ethanol. After dilution with water, these compositions can be used as a mouth wash.

It is now an object of the present invention to provide compositions which exhibit a high level of disinfecting power, which contain relatively low percentages of alcohol and which are free from aldehydes.

It has now been found that the above objects can be met when an anti-microbial compound selected from the group of $C_1$–$C_5$ alkyl esters of p-hydroxybenzoic acid is used in combination with a specific mixture of ethanol and n- propanol.

Accordingly, the present invention provides an aqueous disinfectant composition comprising ethanol and an alkylester or hydroxybenzoic acid, characterized in that it comprises at least 10% by volume of a mixture of ethanol and n-propanol in a volumetric ratio of 1:1 to 1:3 and 0.001 to 10% by weight of a $C_1$–$C_5$ alkyl ester of p-hydroxybenzoic acid.

An essential component of the composition according to the invention is an effective amount of an anti-microbial compound selected from the group of $C_1$–$C_5$ alkyl esters of p-hydroxybenzoic acid. The lower alcohol radical may be derived from $C_1$–$C_5$ alcohols having straight or branched alkyl chains. Particularly effective has been found n-butyl p-hydroxybenzoate, which is therefore the preferred anti-microbial compound to be used in the compositions according to the invention. n-Butyl p-hydroxybenzoate is commercially available under the trade-name Nipabutyl ex Diversey.

The anti-microbial agent is included in an amount which provides effective disinfectant action. Somewhat dependent on the solubility in the aqueous alcohol component, such amount ranges from 0.001% to 10% by weight of the total composition. In particular, such amount ranges from 0.01 to 1% by weight and preferably lies within the range of from 0.05 to 0.3% by weight.

A second essential component of the compositions according to the present invention is a mixture of ethanol and n-propanol. The concentration of the alcohol mixture should be at least 10% v/v and normally lies within the range of from 10% to 50% v/v. Preferred are concentrations which lie within the range of from 20% to 40% v/v. The volume ratio between the ethanol and propanol lies within the range of from 1:1 to 1:3

The composition of the invention further comprises water and, optionally, minor ingredients to improve its effectiveness and/or consumer acceptability. More in particular the composition may contain minor amounts of surfactants, wetting agents, colouring agents and perfumes.

The compositions according to the invention will normally be applied directly by spraying or sprinkling thereof onto the surface to be treated, but they are also particularly suitable for application as the impregnation liquid in tissues which are coming to be known as wet wipes.

The invention will now be further illustrated by way of example.

Example 1

TABLE I

| Organism | Composition (balance water) | | | Log survivors/ ml after 1 hour |
| | % v/v | % w/v | | |
| | ethanol | n-propanol | n-butyl p-hydroxy-benzoate | |
|---|---|---|---|---|
| Staphylococcus | 20[a] | 0 | 0 | 9.3 |
| aureus | 20[a] | 0 | 0.1 | 6.5 |
| | 0[a] | 20 | 0 | 6.7 |
| | 0[a] | 20 | 0.1 | 3.3 |
| | 10[a] | 20 | 0 | 3.3 |
| | 10 | 20 | 0.1 | 2.0 |
| Escherichia | 40[a] | 0 | 0 | 5.8 |
| coli | 40[a] | 0 | 0.5 | 3.0 |
| | 10[a] | 20 | 0 | 4.1 |
| | 10 | 20 | 0.1 | 2.0 |
| Pseudomonas | 10[a] | 20 | 0 | 4.7 |
| aeruginosa | 10 | 20 | 0.1 | 2.0 |
| Candida | 10[a] | 20 | 0 | 2.3 |
| albicans | 10 | 20 | 0.1 | 2.0 |

[a] Comparative examples.

The results in Table I were obtained using the DGHM hard surface disinfection test.

Example 2

| Ingredients | % | |
|---|---|---|
| ethanol | 10 | (v/v) |
| propanol | 20 | (v/v) |
| n-butyl p-hydroxybenzoate | 0.04 | (w/v) |
| water | balance | |

The DGHM suspension disinfection test yielded the following results using Escherichia coli:

| log survivors/ml | after |
|---|---|
| 9.0 | 0 |
| 6.4 | 15 sec. |
| 4.6 | 2 min. |
| 4.0 | 5 min. |
| 3.0 | 10 min. |

Example 3

| Ingredients | % | |
|---|---|---|
| ethanol (96%) | 10 | (v/v) |
| n-propanol | 20 | (v/v) |
| n-butyl p-hydroxybenzoate | 0.1 | (w/v) |
| perfume | 0.05 | (w/v) |
| wetting agent | 0.1 | (w/v) |
| water | balance | |

## Claims

1. Aqueous disinfectant composition comprising ethanol and an alkylester of hydroxybenzoic acid, characterized in that it comprises at least 10% by volume of a mixture of ethanol and n-propanol in a volumetric ratio of 1:1 to 1:3 and 0.001 to 10% by weight of a $C_1–C_5$ alkyl ester of p-hydroxybenzoic acid.

2. Composition according to claim 1, comprising 0.001 to 10% by weight of n-butyl p-hydroxybenzoate.

3. A composition according to claim 2, comprising 0.01 to 1% by weight of n-butyl p-hydroxybenzoate.

4. A composition according to any one of the preceding claims, comprising 10–50% by volume of the mixture of ethanol and n-propanol.

5. A composition according to claim 4, comprising 20–40% by volume of the mixture of ethanol and n-propanol.

6. Wet wipe impregnated with a disinfectant composition according to any one of the preceding claims.

## Patentansprüche

1. Wäßrige Desinfektionsmittel-Zusammensetzung umfassend Ethanol und einen Alkylester von Hydroxybenzoesäure, dadurch gekennzeichnet, daß sie mindestens 10 Volumen-% einer Mischung von Ethanol und n-Propanol in einem volumetrischen Verhältnis von 1:1 bis 1:3 und 0,001 bis 10 Gew.-% eines $C_1–C_5$-Alkylesters von p-Hydroxybenzoesäure umfaßt.

2. Zusammensetzung nach Anspruch 1, umfassend 0,001 bis 10 Gew.-% an n-Butyl-p-hydroxybenzoat.

3. Zusammensetzung nach Anspruch 2, umfassend 0,01 bis 1 Gew.-% an n-Butyl-p-hydroxybenzoat.

4. Zusammensetzung nach einem der vorhergehenden Ansprüche, umfassend 10–50 Volumen-% der Mischung von Ethanol und n-Propanol.

5. Zusammensetzung nach Anspruch 4, umfassend 20–40 Volumen-% der Mischung von Ethanol und n-Propanol.

6. Feucht-Wischtuch imprägniert mit einer Desinfektionsmittel-Zusammensetzung nach einem der vorhergehenden Ansprüche.

## Revendications

1. Composition aqueuse désinfectante comprenant de l'éthanol et un ester alkylique d'acide hydroxybenzoïque, caractérisée en ce qu'elle comprend au moins 10% en volume d'un mélange d'éthanol et de n-propanol dans un rapport volumétrique de 1:1 à 1:3 de 0,001 à 10% en poids d'un ester alkylique en $C_{1-5}$ d'acide p-hydroxybenzoïque.

2. Composition selon la revendication 1, qui comprend de 0,001 à 10% en poids de p-hydroxybenzoate de n-butyle.

3. Composition selon la revendication 2, qui comprend de 0,01 à 1% en poids de p-hydroxybenzoate de n-butyle.

4. Composition selon l'une quelconque des revendications précédentes, qui comprend de 10 à 50% en volume du mélange d'éthanol et de n-propanol.

5. Composition selon la revendication 4, qui comprend de 20 à 40% en volume de mélange d'éthanol et de n-propanol.

6. Serviette mouillée imprégnée d'une composition désinfectante selon l'une quelconque des revendications précédentes.